# EUROPÄISCHE PATENTSCHRIFT

(11) **EP 1 562 540 B1**
(45) Veröffentlichungstag und Bekanntmachung des Hinweises auf die Patenterteilung: **30.08.2006**
(21) Anmeldenummer: 03811732.1
(22) Anmeldetag: 14.05.2003
(51) Int. Cl.: A61Q 5/10, C07D 231/38

(54) **N-ARYL-4,5-DIAMINOPYRAZOLE UND DIESE VERBINDUNGEN ENTHALTENDE FÄRBEMITTEL**
N-ARYL-4,5-DIAMINOPYRAZOLS AND DYES CONTAINING SAID COMPOUNDS
N-ARYL-4,5-DIAMINOPYRAZOLES ET COLORANTS CONTENANT CES COMPOSES

(30) Priorität: 22.11.2002 DE 10254506
(43) Veröffentlichungstag der Anmeldung: 17.08.2005
(73) Patentinhaber: Wella Aktiengesellschaft, 64274 Darmstadt (DE)
(72) Erfinder: GÖTTEL, Otto, CH-1723 Marly (CH); GEIBEL, Wolfram, 36088 Hünfeld (DE); MORAND, Emmanuel, CH-1740 Neyruz (CH)
(86) Internationale Anmeldenummer: PCT/EP2003/005031
(87) Internationale Veröffentlichungsnummer: WO 2004/047781

(56) Entgegenhaltungen:
- EP-A- 0 375 977
- WO-A-03/008405
- DE-U- 20 013 156
- US-A1- 2002 050 013

## Beschreibung

Gegenstand der vorliegenden Anmeldung sind neue, Aryl-substituierte 4,5-Diaminopyrazole sowie diese Verbindungen enthaltende Färbemittel für Keratinfasern.

Auf dem Gebiet der Färbung von Keratinfasern, insbesondere der Haarfärbung, haben Oxidationsfarbstoffe eine wesentliche Bedeutung erlangt. Die Färbung entsteht hierbei durch Reaktion bestimmter Entwicklersubstanzen mit bestimmten Kupplersubstanzen in Gegenwart eines geeigneten Oxidationsmittels. Von besonderer Bedeutung sind nach wie vor Haarfärbemittel zur Färbung im Naturtonbereich. Daneben lassen sich durch Kombination geeigneter Oxidationsfarbstoff-Vorstufen auch zeitgemäß modische Farbnuancen erzeugen. Gegenwärtig im Modetrend liegen abgewandelte Naturtöne wie beispielsweise Brauntöne mit ausgeprägten Aubergine- oder Kupfer-Nuancen, insbesondere aber leuchtende Rottöne.

Neben der Erzeugung von Farbeffekten werden an Oxidationsfarbstoffe, die zur Behandlung menschlicher Haare vorgesehen sind, eine Vielzahl anderer Anforderungen gestellt. So müssen die Farbstoffe in toxikologischer und dermatologischer Hinsicht unbedenklich sein, und die erzielten Haarfärbungen sollen eine gute Lichtechtheit, Dauerwellechtheit, Reibeechtheit und Stabilität gegenüber Schampoonierung sowie eine ausreichende Beständigkeit gegenüber Schweißabsonderungen aufweisen. Außerdem ist es erforderlich, dass durch Kombination geeigneter Entwicklersubstanzen und Kupplersubstanzen eine breite Palette verschiedener Farbnuancen erzeugt werden kann.

Zur Abdeckung des nach wie vor wichtigen Rotbereichs wurde in der Vergangenheit überwiegend 4-Aminophenol als Entwickler verwendet. Wegen Bedenken in Bezug auf die physiologische Verträglichkeit dieser Substanz wurden auch Pyridin- und Pyrimidinderivate eingesetzt, die allerdings in färberischer Hinsicht nicht zufriedenstellen konnten. Eine signifikante Verbesserung der Farbstabilität im Rotbereich wurde erstmals durch den Austausch von p-Aminophenol durch die in der EP-A 0 375 977 beschriebenen 4,5-Diamino-pyrazole erzielt. Weiterhin sind aus der DE-A 101 09 806 Kombinationen von substituierten Pyrazolonen und 4,5-Diamino-pyrazolen zur Erzeugung von gelben bis orangen Nuancen bekannt. Die Verwendung bestimmter 4,5-Diaminopyrazolderivate in Haarfärbemitteln ist auch aus der WO 03/008405 A1, DE 200 13 156 U 1 und US 2002/0050013 A1 bekannt.

Färbemittel für Keratinfasern sind aus der DE 20013156 bekannt.

Während die meisten Oxidationsfarbstoffe auf ungeschädigtem Haar kaum Schwächen zeigen, können sich gravierende Unterschiede auf geschädigtem Haar ergeben. Der Friseur kennt daher aus seiner Alltagspraxis das Problem, dass Farbstoffe nicht gleichmäßig auf das zu färbende Haar aufziehen. Während der Haaransatz in der Regel intakt ist, zeigen im Laufe der Zeit die Haarspitzen infolge von Witterungseinflüssen, häufigem Waschen und Kämmen eine Schädigung, die vom Haaransatz zur Haarspitze graduell zunimmt. Beim Färben solcher Haare kann infolge der ungleichmäßigen Haarbeschaffenheit zwischen Ansatz und Spitzen ein ungleichmäßiges Färbeergebnis erhalten werden. Ein weiteres Problem besteht darin, dass die Farbstoffe beim Waschen aus den stärker geschädigten Haarpartien stärker ausgewaschen werden als aus un-geschädigten Haarpartien, was in Abhängigkeit vom Grad der jeweiligen Haarschädigung, nach einigen Haarwäschen immer deutlicher sichtbar werden kann. Der Kunde bemerkt dies insbesondere auch dadurch, dass die Haarspitzen stumpf aussehen.

Es bestand daher weiterhin ein Bedarf für Farbstoffe, die einerseits sehr brillante Nuancen erzeugen und andererseits über eine erheblich verbesserte Farbstabilität gegenüber Shampoonieren auf den verschiedensten Haarqualitäten, insbesondere auf durch Dauerwellen oder Blondieren geschädigtem Haar, verfügen. Weiterhin sollten die erzielten Farbnuancen ihre Brillanz auch nach mehreren Haarwäschen nicht verlieren.

Es wurde nunmehr gefunden, dass die im folgenden beschriebenen N-Aryl-4,5-diaminopyrazole der Formel (I) die vorgenannten Anforderungen in hervorragender Weise erfüllen, wobei neben einer bisher unerreichten Farbbrillanz und Farbtiefe mit diesen neuen Farbstoffen eine erheblich verbesserte Haltbarkeit erzielt werden kann.

Gegenstand der vorliegenden Erfindung sind daher N-Aryl-4,5-diaminopyrazole der Formel (I) oder deren physiologisch verträglichen Salze mit organischen oder anorganischen Säuren, 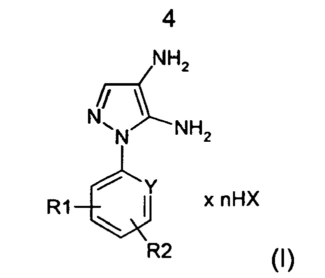 wobei
**R1** und **R2** unabhängig voneinander gleich einer geradkettigen oder verzweigten C1-C6-Alkylgruppe, einer Hydroxygruppe, einer geradkettigen oder verzweigten C1-C6-Monohydroxyalkylgruppe, einer geradkettigen oder verzweigten C3-C6-Dihydroxyalkylgruppe, einer geradkettigen oder verzweigten C1-C6-Alkoxygruppe, einer geradkettigen oder verzweigten C1-C6-Hydroxyalkoxygruppe, einer geradkettigen oder verzweigten C3-C6-Dihydroxyalkoxygruppe, einer Aminogruppe, einer C1-C4-Monoalkylaminogruppe, einer Di(C1-C4)-alkylaminogruppe, einer C1-C4-Aminoalkylgruppe, einem Halogenatom (F, Cl, Br, J), einer Difluormethylgruppe oder einer Trifluormethylgruppe ist;
**Y** eine **C-R3**-Gruppe darstellt, wobei **C** ein Kohlenstoffaton des aromatischen Ringes ist und **R3** gleich einem Wasserstoffatom, einer geradkettigen oder verzweigten C2-C6-Hydroxyalkoxygruppe oder einer geradkettigen oder verzweigten C2-C6-Alkoxyalkoxygruppe ist; und
**X** für einen Säurerest steht und **n** einen Wert von 0 bis 3 hat;
oder **Y** eine **CH**-Gruppe darstellt und **R1** gleich einem Wasserstoffatom ist und **R2** eine Aminogruppe (in 4-Stellung am Benzolring) darstellt und **X** für eine HSO4-Gruppe steht und **n** gleich 1 ist.

Bevorzugt sind Verbindungen der Formel (I) in denen gilt:
**R1** und **R2** stehen unabhängig voneinander für eine Methylgruppe, eine Ethylgruppe, eine Isopropylgruppe, eine Aminogruppe oder eine Methoxygruppe; oder **Y** steht für eine **CH**-Gruppe und **R1** ist gleich einem Wasserstoffatom und **R2** stellt eine Aminogruppe (in 4-Stellung am Benzolring) dar und **X** steht für eine HSO4-Gruppe und **n** ist gleich 1.

Besonders bevorzugt sind die folgenden Verbindungen der Formel (I):

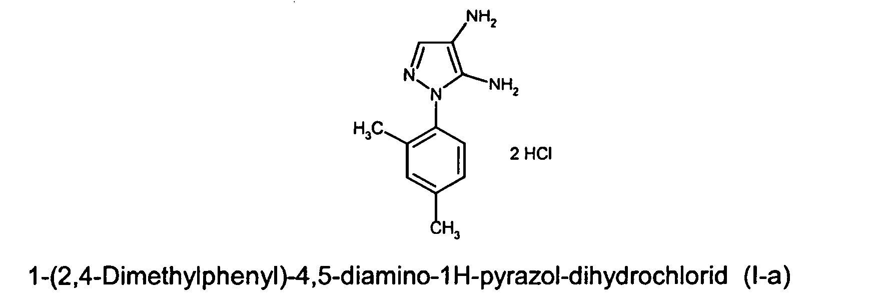

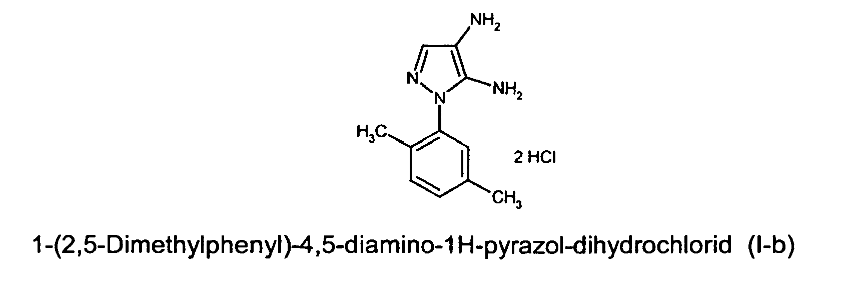

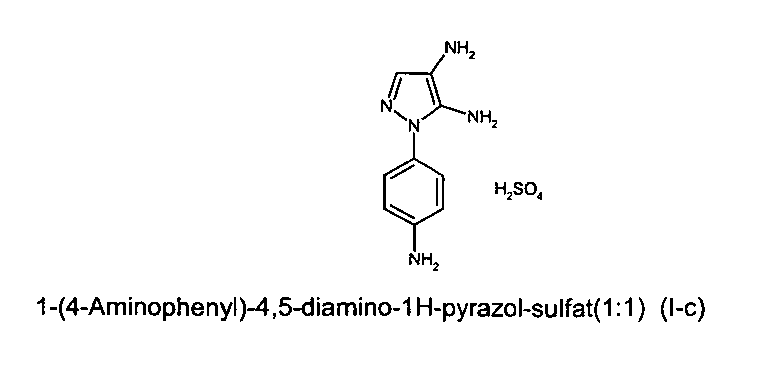

Die Verbindungen der Formel (I) können in Analogie zu bekannten Methoden, beispielsweise durch Zyklisierung eines Arylhydrazins mit Methoxyacrylnitril, Ethoxyacrylnitril oder Chloracrylnitril, oder aber in idealer Weise mit Dimethylaminoacrylnitril nach dem in Schema 1 dargestellten allgemeinen Verfahren, hergestellt werden:

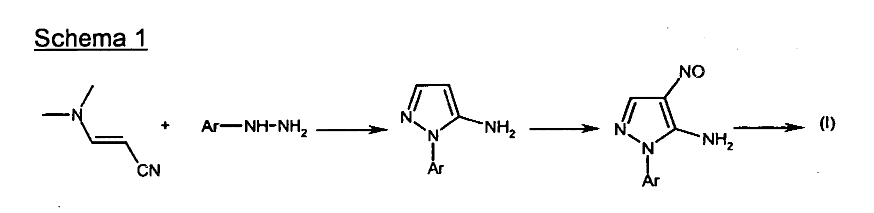

In bestimmten Fällen kann es aber von präparativem Vorteil sein, in der in Schema 2 skizzierten Weise die 5-Aminopyrazole mit einem Diazoniumsalz zunächst zu Azofarbstoffen umzusetzen um nach deren Spaltung die Verbindungen der Formel (I) zu isolieren.

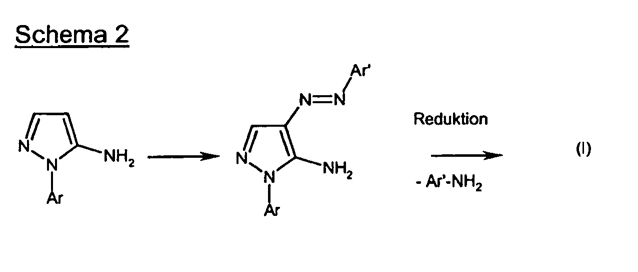

Zur Herstellung der Azo-Zwischenstufen eignen sich in hervorragender Weise Anilin, durch eine oder mehrere C1-C6-Gruppen substituierte Aniline oder Anisidine, Sulfanilsäure, Metanilsäure, Orthanilsäure, p-Aminobenzoesäure, m-Aminobenzoesäure sowie 5-Amino-isophthalsäure. Unter diesen Verbindungen sind Sulfanilsäure, p-Aminobenzoesäure und 5-Amino-isophthalsäure besonders bevorzugt.

Die erfindungsgemäßen N-Aryl-4,5-diaminopyrazole der Formel (I) werden zwecks besserer Handhabung nicht in Form ihrer freie Basen, sondern vorzugsweise in Form der oxidationsunempfindlicheren entsprechenden Salze isoliert. Als Säuren können hierbei anorganische oder organische Säuren eingesetzt werden, wobei Zitronensäure, Weinsäure, Phosphorsäure und insbesondere Salzsäure oder Schwefelsäure bevorzugt sind.

Die Verbindungen der Formel (I) eignen sich hervorragend als Farbstoff-Vorstufen im oxidativen System zum Färben von Keratinfasern. Obwohl sich die Verbindungen insbesondere für die Verwendung zur Färbung von Keratinfasern, beispielsweise Wolle, Seide oder Haaren -insbesondere menschliche Haare-, eignen, ist es prinzipiell auch möglich, mit diesen Verbindungen andere natürliche oder synthetische Fasern, beispielsweise Baumwolle oder Nylon 66, zu färben.

Ein weiterer Gegenstand der vorliegenden Erfindung ist daher ein Mittel zur oxidativen Färbung von Keratinfasern, wie zum Beispiel Wolle, Pelzen, Federn oder Haaren und insbesondere menschlichen Haaren, welches dadurch gekennzeichnet ist, dass es mindestens ein N-Aryl-4,5-diaminopyrazol der allgemeinen Formel (I) oder dessen physiologisch verträgliches Salz enthält.

Die N-Aryl-4,5-diaminopyrazole der Formel (I) sind in dem erfindungsgemäßen Färbemittel in einer Menge von etwa 0,005 bis 20 Gewichtsprozent enthalten, wobei eine Menge von etwa 0,01 bis 10 Gewichtsprozent und insbesondere 0,1 bis 6 Gewichtsprozent bevorzugt ist.

Die Verbindungen der Formel (I) können sowohl alleine als auch in Kombination mit bekannten Entwicklersubstanzen und/oder Kupplersubstanzen, die üblicherweise in oxidativen Färbesystemen zur Färbung von Fasermaterialien Verwendung finden, eingesetzt werden.

Als geeignete Kupplersubstanzen können insbesondere genannt werden: N-(3-Dimethylamino-phenyl)-harnstoff, 2,6-Diamino-pyridin, 2-Amino-4-[(2-hydroxyethyl)amino]-anisol, 2,4-Diamino-1-fluor-5-methyl-benzol, 2,4-Diamino-1-methoxy-5-methyl-benzol, 2,4-Diamino-1-ethoxy-5-methylbenzol, 2,4-Diamino-1-(2-hydroxyethoxy)-5-methyl-benzol, 2,4-Di[(2-hydroxyethyl)amino]-1,5-dimethoxy-benzol, 2,3-Diamino-6-methoxypyridin, 3-Amino-6-methoxy-2-(methylamino)-pyridin, 2,6-Diamino-3,5-dimethoxy-pyridin, 3,5-Diamino-2,6-dimethoxy-pyridin, 1,3-Diamino-benzol, 2,4-Diamino-1-(2-hydroxyethoxy)-benzol, 2,4-Diamino-1-(3-hydroxypropoxy)-benzol, 2,4-Diamino-1-(3-methoxypropoxy)-benzol, 1-(2-Aminoethoxy)-2,4-diamino-benzol, 2-Amino-1-(2-hydroxyethoxy)-4-methylamino-benzol, 2,4-Diaminophenoxy-essigsäure, 3-[Di(2-hydroxyethyl)amino]-anilin, 4-Amino-2-di[(2-hydroxyethyl)amino]-1-ethoxy-benzol, 5-Methyl-2-(1-methylethyl)-phenol, 3-[(2-Hydroxyethyl)amino]-anilin, 3-[(2-Aminoethyl)-amino]-anilin, 1,3-Di(2,4-diaminophenoxy)-propan, Di(2,4-diaminophenoxy)-methan, 1,3-Diamino-2,4-dimethoxy-benzol, 2,6-Bis(2-hydroxyethyl)amino-toluol, 4-Hydroxyindol, 3-Dimethylamino-phenol, 3-Diethylamino-phenol, 5-Amino-2-methyl-phenol, 5-Amino-4-fluor-2-methyl-phenol, 5-Amino-4-methoxy-2-methyl-phenol, 5-Amino-4-ethoxy-2-methyl-phenol, 3-Amino-2,4-dichlor-phenol, 5-Amino-2,4-dichlor-phenol, 3-Amino-2-methyl-phenol, 3-Amino-2-chlor-6-methyl-phenol, 3-Aminophenol, 2-[(3-Hydroxyphenyl)-amino]-acetamid, 5-[(2-Hydroxyethyl)-amino]-2-methyl-phenol, 3-[(2-Hydroxy-ethyl)amino]-phenol, 3-[(2-Methoxyethyl)amino]-phenol, 5-Amino-2-ethyl-phenol, 2-(4-Amino-2-hydroxyphenoxy)-ethanol, 5-[(3-Hydroxypropyl)amino]-2-methyl-phenol, 3-[(2,3-Dihydroxypropyl)-amino]-2-methyl-phenol, 3-[(2-Hydroxyethyl)-amino]-2-methyl-phenol, 2-Amino-3-hydroxy-pyridin, 5-Amino-4-chlor-2-methyl-phenol, 1-Naphthol, 1,5-Dihydroxy-naphthalin, 1,7-Dihydroxy-naphthalin, 2,3-Dihydroxy-naphthalin, 2,7-Dihydroxy-naphthalin, 2-Methyl-1-naphthol-acetat, 1,3-Dihydroxy-benzol, 1-Chlor-2,4-dihydroxy-benzol, 2-Chlor-1,3-dihydroxy-benzol, 1,2-Dichlor-3,5-dihydroxy-4-methyl-benzol, 1,5-Dichlor-2,4-dihydroxy-benzol, 1,3-Dihydroxy-2-methyl-benzol, 1,3-Dihydroxy-2,4-dimethyl-benzol, 3,4-Methylendioxy-phenol, 3,4-Methylendioxy-anilin, 5-[(2-Hydroxyethyl)amino]-1,3-benzodioxol, 6-Brom-1-hydroxy-3,4-methylendioxy-benzol, 3,4-Diamino-benzoesäure, 3,4-Dihydro-6-hydroxy-1,4(2H)-benzoxazin, 6-Amino-3,4-dihydro-1,4(2H)-benzoxazin, 3-Methyl-1-phenyl-5-pyrazolon, 5,6-Dihydroxy-indol, 5,6-Dihydroxy-indolin, 4-Hydroxy-indol, 5-Hydroxy-indol, 6-Hydroxy-indol, 7-Hydroxy-indol und 2,3-Indolindion, oder deren Salze.

Zur Herstellung von naturnahen Tönen und modischen Rottönen ist es besonders vorteilhaft, Verbindungen der Formel (I) in Kombination mit zusätzlichen Entwicklersubstanzen einzusetzen. Als Entwicklersubstanzen kommen p-Phenylendiamine, p-Aminophenole sowie weitere 4,5-Diaminopyrazole, oder deren Salze in Betracht. Insbesondere sind die folgenden Entwicklersubstanzen zu nennen: 1,4-Diamino-benzol (p-Phenylendiamin), 1,4-Diamino-2-methyl-benzol (p-Toluylendiamin), 1,4-Diamino-2,6-dimethyl-benzol, 1,4-Diamino-2,5-dimethyl-benzol, 1,4-Diamino-2,3-dimethyl-benzol, 2-Chlor-1,4-diamino-benzol, 4-Phenylamino-anilin, 4-Dimethylamino-anilin, 4-Diethylaminoanilin, 4-[Di(2-hydroxyethyl)amino]-anilin, 4-[(2-Methoxyethyl)amino]-anilin, 4-[(3-Hydroxypropyl)amino]-anilin, 1,4-Diarnino-2-(2-hydroxyethyl)-benzol, 1,4-Diamino-2-(1-hydroxyethyl)-benzol, 1,4-Diamino-2-(1-methylethyl)-benzol, 1,3-Bis[(4-aminophenyl)-(2-hydroxyethyl)amino]-2-propanol, 1,8-Bis(2,5-diaminophenoxy)-3,6-dioxaoctan, 4-Amino-phenol, 4-Amino-3-methyl-phenol, 4-Methylamino-phenol, 4-Amino-2-(aminomethyl)-phenol, 4-Amino-2-[(2-hydroxyethyl)-amino]methyl-phenol, 4-Amino-2-(methoxymethyl)-phenol, 4-Amino-2-(2-hydroxyethy)-phenol, 5-Amino-salicylsäure, 2,5-Diamino-pyridin, 2,4,5,6-Tetraamino-pyrimidin, 2,5,6-Triamino-4-(1 H)-pyrimidon, 4,5-Diamino-1-(2-hydroxyethyl)-1H-pyrazol, 4,5-Diamino-1-(1-methylethyl)-1H-pyrazol, 4,5-Diamino-1-(4-methylbenzyl)-1H-pyrazol, 1-(4-Chlorbenzyl)-4,5-diamino-1 H-pyrazol, 4,5-Diamino-1-methyl-1 H-pyrazol, 4,5-Diamino-3-methyl-1-phenyl-1H-pyrazol, 4,5-Diamino-1-methyl-1 H-pyrazol, 4,5-Diamino-1-pentyl-1 H-pyrazol, 4,5-Diamino-1-benzyl-1 H-pyrazol, 4,5-Diamino-1-(4-methoxybenzyl)-1 H-pyrazol, 4,5-Diamino-1-(2-hydroxyethyl)-3-methyl-1H-pyrazol, 2-Amino-phenol, 2-Amino-6-methyl-phenol und 2-Amino-5-methyl-phenol oder deren Salze.

Die vorgenannten Entwicklersubstanzen und Kupplersubstanzen können jeweils einzeln oder im Gemisch miteinander verwendet werden, wobei die vorgenannten bekannten Entwicklersubstanzen und Kupplersubstanzen in dem erfindungsgemäßen Färbemittel jeweils in einer Gesamtmenge von etwa 0,01 bis 20 Gewichtsprozent, vorzugsweise etwa 0,2 und 6 Gewichtsprozent, enthalten sind.

Weiterhin kann das erfindungsgemäße Färbemittel zusätzlich andere Farbkomponenten, beispielsweise 4-(2,5-Diamino-benzylamino)-anilin oder 3-(2,5-Diamino-benzylamino)-anilin, sowie ferner übliche natürliche, naturidentische oder synthetische direktziehende Farbstoffe aus der Gruppe der anionischen (sauren) und kationischen (basischen) Farbstoffe, der Triarylmethanfarbstoffe, der Nitrofarbstoffe, der Dispersionsfarbstoffe und der Azofarbstoffe enthalten, zum Beispiel natürliche Farbstoffe wie Indigo oder Henna, Triphenylmethanfarbstoffe wie 4-[(4'-amino-phenyl)-(4'imino-2",5"-cyclohexadien-1 "-yliden)-methyl]-2-methyl-aminobenzol-monohydrochlorid (C.l. 42 510) und 4-[(4'-amino-3'-methyl-phenyl)-(4"-imino-3"-methyl-2",5"cyclohexadien-1"-yliden)-methyl]-2-methyl-aminobenzol monohydrochlorid (C.l. 42 520), aromatische Nitrofarbstoffe wie 4-(2'-hydroxyethyl)amino-nitrotoluol, 2-Amino-4,6-dinitrophenol, 2-Amino-5-(2'-hydroxyethyl)amino-nitrobenzol, 2-Chlor-6-(ethylamino)-4-nitrophenol, 4-Chlor-N-(2-hydroxyethyl)-2-nitroanilin, 5-Chlor-2-hydroxy-4-nitroanilin, 2-Amino-4-chlor-6-nitrophenol und 1-[(2'-Ureidoethyl)amino-4-nitrobenzol, Azofarbstoffe wie 6-[(4'-Aminophenyl)-azo]-5hydroxy-naphthalin-1-sulfonsäure-Natriumsalz (C.l. 14 805) und Dispersionsfarbstoffe wie beispielsweise 1,4-Diaminoanthrachinon und 1,4,5,8-Tetraaminoantrachinon.

Die Gesamtkonzentration an direktziehenden Farbstoffen beträgt in dem erfindungsgemäßen Mittel etwa 0,1 bis 10 Gewichtsprozent, vorzugsweise etwa 0,1 bis 5 Gewichtsprozent.

Selbstverständlich können die Farbstoffe, sofern es Basen sind, auch in Form ihrer physiologisch verträglichen Salze mit organischen oder anorganischen Säuren, wie beispielsweise Salzsäure oder Schwefelsäure, beziehungsweise - sofern sie aromatische OH-Gruppen besitzen - in Form der Salze mit Basen, zum Beispiel als Alkaliphenolate, eingesetzt werden.

Die vorstehend beschriebenen erfindungsgemäßen Kombinationen der Verbindungen der Formel (I) mit oxidativen Haarfarbvorstufen und/oder direktziehenden Farbstoffen werden zur Färbung in einer geeigneten Farbträgermasse appliziert.

Darüber hinaus können in dem Färbemittel noch weitere übliche Zusatzstoffe, beispielsweise Antioxidantien wie Ascorbinsäure, Thioglykolsäure oder Natriumsulfit, sowie Parfümöle, Penetrationsmittel, Puffersysteme, Komplexbildner, Konservierungsstoffe, Netzmittel, Emulgatoren, Verdicker und Pflegestoffe enthalten sein.

Die Zubereitungsform des erfindungsgemäßen Färbemittels kann beispielsweise eine Lösung, insbesondere eine wässrige oder wässrigalkoholische Lösung, eine Paste, eine Creme, ein Gel, eine Emulsion oder eine Aerosolzubereitung sein. Ihre Zusammensetzung stellt eine Mischung der Farbstoffkomponenten mit den für solche Zubereitungen üblichen Zusätzen dar.

Ubliche Zusätze in Lösungen, Cremes, Emulsionen oder Gelen sind zum Beispiel Lösungsmittel wie Wasser, niedere aliphatische Alkohole, beispielsweise Ethanol, Propanol oder Isopropanol, Glycerin oder Glykole wie 1,2-Propylenglykol, weiterhin Netzmittel oder Emulgatoren aus den Klassen der anionischen, kationischen, amphoteren oder nichtionogenen oberflächenaktiven Substanzen wie zum Beispiel Fettalkoholsulfate, oxethylierte Fettalkoholsulfate, Alkylsulfonate, Alkylbenzolsulfonate, Alkyltrimethylammoniumsalze, Alkylbetaine, oxethylierte Fettalkohole, oxethylierte Nonylphenole, Fettsäurealkanolamide und oxethylierte Fettsäureester, ferner Verdicker wie höhere Fettalkohole, Stärke, Cellulosederivate, Petrolatum, Paraffinöl und Fettsäuren, sowie außerdem Pflegestoffe wie kationische Harze, Lanolinderivate, Cholesterin, Pantothensäure und Betain. Die erwähnten Bestandteile werden in den für solche Zwecke üblichen Mengen verwendet, zum Beispiel die Netzmittel und Emulgatoren in Konzentrationen von etwa 0,5 bis 30 Gewichtsprozent, die Verdicker in einer Menge von etwa 0,1 bis 30 Gewichtsprozent und die Pflegestoffe in einer Konzentration von etwa 0,1 bis 5 Gewichtsprozent.

Je nach Zusammensetzung kann das erfindungsgemäße Färbemittel schwach sauer, neutral oder alkalisch reagieren. Insbesondere weist es einen pH-Wert von 6 bis 11,5 auf, wobei die basische Einstellung vorzugsweise mit Ammoniak oder organischen Aminen, zum Beispiel Monoethanolamin und Triethanolamin, oder aber Aminosäuren oder anorganischen Basen wie Natriumhydroxid und Kaliumhydroxid, erfolgt. Ebenfalls ist es möglich, Kombinationen der vorgenannten Verbindungen, insbesondere eine Kombinbation von Ammoniak und Monoethanolamin, zu verwenden. Für eine pH-Einstellung im sauren Bereich kommen anorganische oder organische Säuren, zum Beispiel Phosphorsäure, Essigsäure Zitronensäure oder Weinsäure, in Betracht.

Für die Anwendung zur oxidativen Färbung von Haaren vermischt man das vorstehend beschriebene Färbemittel (pH = 6 bis 11,5) unmittelbar vor dem Gebrauch mit einem Oxidationsmittel (pH = 2 bis 6,5). Der pH-Wert des gebrauchsfertigen Haarfärbemittels stellt sich hierbei auf einen pH-Wert ein, der durch die Alkalimenge in der Farbträgermasse und die Säuremenge im Oxidationsmittel sowie durch das Mischungsverhältnis bestimmt wird. Je nach Zusammensetzung kann das gebrauchsfertige Färbemittel schwach sauer, neutral oder alkalisch reagieren und einen pH-Wert von etwa 3 und 11, vorzugsweise etwa 5 bis 10, aufweisen.

Als Oxidationsmittel zur Entwicklung der Haarfärbung kommen hauptsächlich Wasserstoffperoxid oder dessen Additionsverbindungen an Harnstoff, Melamin, Natriumborat oder Natriumcarbonat in Form einer 3-bis 12prozentigen, vorzugsweise 6prozentigen, wässrigen Lösung, aber auch Luftsauerstoff in Betracht. Bezogen auf einen Gehalt an freiem Wasserstoffperoxid von 6 Prozent im Oxidationsmittel beträgt das Gewichtsverhältnis zwischen Haarfärbemittel und Oxidationsmittel etwa 5:1 bis 1:2, vorzugsweise jedoch 1:1. Größere Mengen an Oxidationsmittel werden vor allem bei höheren Farbstoffkonzentrationen im Haarfärbemittel, oder wenn gleichzeitig eine stärkere Bleichung des Haares beabsichtigt ist, verwendet.

Man trägt eine für die Haarfärbebehandlung ausreichende Menge, je nach Haarfülle, im allgemeinen etwa 60 bis 200 Gramm, dieses Gemisches auf das Haar auf und läßt das Gemisch bei 15 bis 50 Grad Celsius etwa 10 bis 45 Minuten lang, vorzugsweise 30 Minuten lang, auf das Haar einwirken, spült sodann das Haar mit Wasser aus und trocknet es. Gegebenenfalls wird im Anschluß an diese Spülung mit einem Shampoo gewaschen und falls erforderlich mit einer schwachen organischen Säure, wie zum Beispiel Zitronensäure oder Weinsäure, nachgespült. Anschließend wird das Haar getrocknet.

Die erfindungsgemäßen Färbemittel mit einem Gehalt an N-Aryl-4,5-diaminopyrazolen der Formel (I) ermöglichen Haarfärbungen mit ausgezeichneter Farbechtheit, insbesondere was die Lichtechtheit, Waschechtheit und Reibeechtheit anbetrifft. Hinsichtlich der färberischen Eigenschaften bieten die erfindungsgemäßen Haarfärbemittel je nach Art und Zusammensetzung der Farbkomponenten eine breite Palette verschiedener Farbnuancen, insbesondere im Bereich der modischen Rottöne. Hierbei zeichnen sich die Farbtöne durch ihre besondere Farbintensität und Leuchtkraft aus. Die sehr guten färberischen Eigenschaften der Färbemittel gemäß der vorliegenden Anmeldung zeigen sich insbesondere darin, dass diese Mittel auch unterschiedlich stark vorgeschädigtem Haar eine gleichmässige und haltbare Anfärbung ermöglichen.

Die nachfolgenden Beispiele sollen den Gegenstand der Erfindung näher erläutern, ohne ihn darauf zu beschränken.

### Beispiele

### Beispiel 1: Herstellung von 1-(4-Methoxyphenyl)-4,5-diamino-1H-pyrazol-dihydrochlorid

### Stufe 1.1: 5-Aminol -(4-methoxyphenyl)-1 H-pyrazol

20,43 g (117 mmol) 4-Methoxyphenylhydrazin-hydrochlorid und 12,5 g (130 mmol) 3-Dimethylamino-acrylnitril werden in 200 ml Methanol 2 Stunden lang unter Rückfluß erhitzt. Anschließend läßt man die Reaktionsmischung abkühlen und gießt auf 600 ml Eiswasser, wobei das 5-Amino-1-(4-methoxyphenyl)-pyrazol ausfällt. Abfiltrieren und Trocknen ergeben 17,4 g (92 mmol, 79 % der Theorie) eines violettstichigen Produktes.
¹H-NMR (DMSO-*d*_{*6*}): δ= 3,60 ppm (d, ⁴J_{HH} = 7,0 Hz, 2H); 3,67 ppm (s, 3H); 6,81 ppm (d, ³J_{HH} = 11,0 Hz, 2H); 6,85 ppm (d, ³J_{HH} = 11,0 Hz, 2H); 7,00 ppm (t, ⁴J_{HH} = 7,0 Hz, 1 H); 9,90 ppm (s, 1 H).

### Stufe 1.2: 5-Amino-1-(4-methoxyphenyl)-4-nitroso-pyrazol-hydrochlorid

17,4 g (92 mmol) 5-Amino-1-(4-methoxyphenyl)-pyrazol aus Stufe 1.1 werden in 200 ml Tetrahydrofuran gelöst, mit 52,4 g 32%iger Salzsäure versetzt und auf 0 bis 5 °C gekühlt. Anschließend werden unter Rühren 11,8 g (101 mmol) Isopentylnitrit so zugetropft, dass die Temperatur 5 °C nicht übersteigt. Es bildet sich allmählich ein bräunlicher Niederschlag, der nach 2stündigem Weiterrühren im Eisbad abgesaugt und mit wenig Tetrahydrofuran nachgewaschen wird. Nach dem Trocknen erhält man 16,4 g (64 mmol, 70 % der Theorie) 5-Amino-1-(4-methoxyphenyl)-4-nitroso-pyrazol-hydrochlorid.
¹H-NMR (DMSO-*d*_{*6*}): δ= 3,83 ppm (s, 3H); 7,0 ppm (s breit, 3H); 7,11 ppm (d, ³J_{HH} = 15 Hz, 2H); 7,45 ppm (d, ³J_{HH} = 15 Hz, 2H); 8,75 ppm (s, 1 H).

### Stufe 1.3: 1-(4-Methoxyphenyl)-4,5-diamino-1H-pyrazoldihydrochlorid

8,0 g (31 mmol) 5-Amino-1-(4-methoxyphenyl)-4-nitroso-pyrazol-hydrochlorid aus Stufe 1.2 werden in 200 ml Ethanol 6 Stunden lang an 0,8 g Pd/C (10%ig) bei 8 bar Wasserstoffdruck hydriert. Der Katalysator wird abfiltriert und den Reaktionsansatz auf etwa 30 ml Gesamtvolumen aufkonzentriert. Nach Versetzten mit 40 ml einer 3molaren ethanolischen Salzsäure und Rühren im Eisbad kristallisiert das Produkt aus. Absaugen und Nachwaschen mit 50 ml Essigsäureethylester liefert 7,4 g (27 mmol, 86 % der Theorie) 1-(4-Methoxyphenyl)-4,5-diamino-1 H-pyrazoldihydrochlorid.
¹H-NMR (DMSO-*d*_{*6*}): δ = 3,82 ppm (s, 3H); 5,29 ppm (s breit, 3H); 7,07 ppm (d, ³J_{HH} = 15 Hz, 2H); 7,44 ppm (d, ³J_{HH} = 15 Hz, 2H); 7,46 ppm (s, 1 H); 10,04 ppm (s breit, 3H).

### Beispiel 2: Herstellung von 1-(4-Isopropylphenyl)-4,5-diamino-1H-pyrazol-dihydrochlorid

### Stufe 2.1: 5-Amino-1-(4-isopropylphenyl)-1H-pyrazol

15,7 g (84 mmol) 4-Isopropyl-phenylhydrazin-hydrochlorid und 8,9 g (93 mmol) 3-Dimethylamino-acrylnitril werden in 150 ml Methanol 3 Stunden lang unter Rückfluß erhitzt. Anschließend läßt man abkühlen und gießt auf 600 ml Eiswasser, wobei das 5-Amino-1-(4-isopropylphenyl)-pyrazol ausfällt. Abfiltrieren, nachwaschen mit 100 ml Wasser und Trocknen ergeben 14,9 g (74 mmol, 88 % der Theorie) beiges Produkt.
¹H-NMR (DMSO-*d*_{*6*}): δ= 1,17 ppm (d, ³J_{HH} = 11,3 Hz, 6H); 2,78 ppm (h, ³J_{HH} = 11,3 Hz, 1 H); 6,88 ppm (d, ³J_{HH} = 14,1 Hz, 2H); 7,05 ppm (s, 1 H); 7,07 ppm (d, ³J_{HH} = 14,1 Hz, 2H); 10,02 ppm (s, 1H).

### Stufe 2.2: 5-Amino-1-(4-isopropylphenyl)-4-nitroso-1H-pyrazol-hydrochlorid

14,9 g (74 mmol) 5-Amino-1-(4-isopropylphenyl)-pyrazol aus Stufe 2.1 werden in 130 ml Tetrahydrofuran gelöst, mit 42,2 g 32%iger Salzsäure versetzt und auf 0 bis 5 °C gekühlt. Anschließend werden unter Rühren 9,5 g (81 mmol) Isopentylnitrit so zugetropft, dass die Temperatur 5 °C nicht übersteigt. Es bildet sich allmählich ein gelblicher Niederschlag, der nach 1,5stündigem Weiterrühren im Eisbad abgesaugt und mit wenig Tetrahydrofuran nachgewaschen wird. Nach dem Trocknen erhält man 12,3 g (46,1 mmol, 62 % der Theorie) 5-Amino-1-(4-isopropylphenyl)-4-nitroso-1 H-pyrazol-hydrochlorid.
¹H-NMR (DMSO-*d*_{*6*}): δ= 1,25 ppm (d, ³J_{HH} = 11,7 Hz, 6H); 2,99 ppm (h, ³J_{HH} = 11,7 Hz, 1 H); 7,45 ppm (m zentriert, 4H); 8,77 ppm (s, 1 H).

### Stufe 2.3: 4,5-Diamino-1-(4-isopropylphenyl)-1H-pyrazoldihydrochlorid

6,0 g (22,5 mmol) 5-Amino-1-(4-isopropylphenyl)-4-nitroso-1 H-pyrazol-hydrochlorid aus Stufe 2.2 werden in 150 ml Ethanol 6 Stunden lang an 0,6 g Pd/C (10%ig) bei 8 bar Wasserstoffdruck hydriert. Der Katalysator wird abfiltriert und das Filtrat auf etwa 20 ml Gesamtvolumen aufkonzentriert. Nach Versetzten mit 40 ml einer 3molaren ethanolischen Salzsäure und Rühren im Eisbad kristallisiert das Produkt aus. Absaugen und Nachwaschen mit 50 ml Essigsäureethylester liefert 5 g (17,2 mmol, 77% der Theorie) 4,5-Diamino-1-(4-isopropylphenyl)-1 H-pyrazoldihydrochlorid als farbloses Produkt.
¹H-NMR (DMSO-*d*_{*6*}): δ= 1,24 ppm (d, ³J_{HH} = 11 Hz, 6H); 2,97 ppm (h, ³J_{HH} = 11 Hz, 1 H); 7,39 ppm (d, ³J_{HH} = 14,2 Hz, 2H); 7,47 ppm (d, ³J_{HH} = 14,2 Hz, 2H); 7,49 ppm (s, 1 H); 7,61 ppm (s breit, 3H); 10,15 ppm (s breit, 3H).

Die in den nachfolgenden Beispielen 3 und 4 beschriebenen N-Aryl-4,5-diaminopyrazole können in Analogie zu den Beispielen 1 und 2 unter Verwendung der entsprechenden Hydrazine hergestellt werden.

### Beispiel 3: 1-(2,4-Dimethylphenyl)-4,5-diamino-1H-pyrazoldihydrochlorid

¹H-NMR (DMSO-*d*_{*6*}): δ= 1,99 ppm (s, 3H); 2,32 ppm (s, 3H); 4,49 ppm (s breit, 3H); 7,14 ppm (m zentriert, 2H); 7,22 ppm (s, 1 H); 7,49 ppm (s, 1 H); 10,04 ppm (s breit, 3H).

### Beispiel 4: 1-(2,5-Dimethylphenyl)-4,5-diamino-1H-pyrazoldihydrochlorid

¹H-NMR (DMSO-*d*_{*6*}): δ= 2,00 ppm (s, 3H); 2,32 ppm (s, 3H); 4,49 ppm (s breit, 3H); 7,07 ppm (s, 1 H); 7,10-7,30 ppm (m, 2H); 7,46 ppm (s, 1 H); 10,11 ppm (s breit, 3H).

### Beispiel 5: 1-(4-Aminophenyl)-4,5-diamino-1 H-pyrazol-sulfat

### Stufe 5.1: 5-Amino-1-(4-nitrophenyl)-1H-pyrazol

7,65 g (50 mmol) 4-Nitrophenylhydrazin werden in 70 ml Wasser und 10 ml n-Propanol suspendiert und auf zirka 50 °C erwärmt. Bei dieser Temperatur werden anschließend 5 g (50 mmol) konzentrierte Salzsäure und 4,81 g (50 mmol) 3-Dimethylamino-acrylnitril zugegeben. Nach weiteren 15 Minuten werden innerhalb von 15 Minuten 3,8 ml (50 mmol) einer 25%igen Ammoniaklösung zugetropft. Sodann wird die Reaktionsmischung bis zur vollständigen Umsetzung des 3-Dimethylamino-acrylnitril bei zirka 50 °C weitergerührt (Dauer etwa 1 Stunde) und anschließend die entstandene dunkelbraune Suspension im Eisbad abgekühlt. Der Niederschlag wird filtriert, 3mal mit wenig kaltem Wasser gewaschen und sodann getrocknet. Rohausbeute: 9,4 g (46 mmol, 92 % der Theorie).
Das erhaltene Rohprodukt kann ohne weitere Reinigung für in Stufe 11.2 verwendet werden. Eine für analytische Zwecke aus Acetonitril/ Wasser 1:1 umkristallisierte Probe hatte einen Schmelzpunkt von 166,7 °C.

### Stufe 5.2: 5-Amino-1-(4-nitrophenyl)-4-(4-sulfophenylazo)-pyrazol

7,79 g (45 mmol) Sulfanilsäure werden diazotiert, mit 7,38 g (90 mmol) Natriumacetat versetzt und noch zirka 15 Minuten lang bei 0 bis 5°C weitergerührt. Anschließend werden 9,2 g (45 mmol) 5-Amino-1-(4-nitrophenyl)-1 H-pyrazol aus Stufe 11.1 (gelöst in 20 ml Dimethylformamid) innnerhalb von 30 Minuten zugetropft. Im Verlauf des Zutropfens färbt sich die Reaktiosmischung gelbbraun und man erhält eine zunehmend dicker werdende Suspension. Man verdünnt die Suspension mit zirka 50 ml kaltem Wasser und läßt die Reaktionsmischung über Nacht bei Raumtemperatur stehen. Anschließend wird der Niederschlag abgesaugt und mit wenig kaltem Wasser nachgewaschen. Das so erhaltene feuchte Rohprodukt (etwa 14 g) wird anschließend in 100 ml Isopropanol suspendiert, mit 10 ml (72 mmol) Triethylamin versetzt und unter Rückfluß erhitzt. Nach zirka 30 Minuten wird die Reaktionsmischung leicht abgekühlt und tropfenweise mit 14,5 ml (112 mmol) einer 25%igen Salzsäurelösung versetzt, wobei eine dicke, ockergelbe Suspension entsteht. Man kühlt auf 0 bis 5 °C ab, saugt den Niederschlag ab und wäscht mit wenig Essigsäureethylester nach. Der Rückstand wird bei 60 °C im Vakuum getrocknet. Es werden 12,18 g (31,4 mmol, 69,7 % der Theorie) 5-Amino-1-(4-nitrophenyl)-4-(4-sulfophenylazo)-pyrazol mit Schmelzpunkt 135-136 °C (Zers.) erhalten.

### Stufe 5.3: 1-(4 Aminophenyl)-4,5-diaminopyrazol-sulfat

11,65 g (30 mmol) 5-Amino-1-(4-nitrophenyl)-4-(4-sulfophenylazo)-pyrazol aus Stufe 11.2 werden in 140 ml Methanol suspendiert und an 0,3 g Pd/C (10%ig) bei 60 °C unter einem Wasserstoffvordruck von zirka 2 bar hydriert. Nach etwa 1 Stunde ist die Umsetzung beendet und der Ansatz wird auf Raum-temperatur abgekühlt. Unter Stickstoff wird der Katalysator abfiltriert und das Filtrat in Gegenwart von 22,2 g (etwa 32 mmol) Ionenaustauscher Amberlyst A 26 (stark basisch) solange gerührt, bis chromatographisch keine Sulfanilsäure mehr in der Lösung nachweisbar ist. Anschließend wird vom Ionenaustauscher unter Stickstoff abfiltriert und 3mal mit je 30 ml Methanol nachgewaschen. Das Filtrat wird anschließend sofort unter Rühren bei Raumtemperatur in eine Mischung aus 3,8 g (39 mmol) konzentrierter Schwefelsäure und 12 ml Methanol getropft. Die gebildete Suspension wird abgesaugt, mit wenig Methanol gewaschen und im Vakuum bei 60 °C getrocknet. Es werden 6,67 g (23,2 mmol, 77,4 % der Theorie) Rohprodukt erhalten.
Zur Reinigung werden unter Stickstoff 1,30 g (4,5 mmol) des Rohproduktes in 10 ml Isopropanol und 2,5 ml Wasser suspendiert und nach Zugabe von 1,4 ml (10 mmol) Triethylamin unter Erwärmen auf zirka 35 °C gelöst und mit 0,1 g Aktivkohle 15 Minuten lang gerührt. Anschließend wird die Aktivkohle abgesaugt und die Lösung bei Raumtemperatur langsam mit 0,3 ml (5,5 mmol) konzentrierter Schwefelsäure versetzt, wobei ein rötlicher Niederschlag ausfällt. Der Niederschlag wird abfiltriert, mit wenig Isopropanol nachgewaschen und im Vakuum bei 60 °C getrocknet. Es werden 1,1 g (3,8 mmol, 85 % der Theorie) 1-(4 Aminophenyl)-4,5-diaminopyrazol-sulfat erhalten.
¹H-NMR (DMSO-*d*_{*6*}): δ= 3,52 ppm (s sehr breit, 6H); 5,23 ppm (s sehr breit, 2H); 6,66 ppm (d, ³J_{HH} = 14,4 Hz, 2H); 7,10 ppm (d, ³J_{HH} = 14,4 Hz, 2H); 7,37 ppm (s, 1 H).
Elementaranalyse: C₉H₁₁N₅ x½ H₂SO₄ (M = 257,69)

| | C | H | N | S |
|---|---|---|---|---|
| berechnet : | 37,64 % | 4,56% | 24,38 % | 11,14 % |
| gefunden : | 37,76 % | 4,67 % | 24,23 % | 11,02 % |

### Beispiel 6: Oxidationshaarfärbemittel, basisch

| | |
|---|---|
| 0,30 g | Ascorbinsäure |
| 0,40 g | Natriumsulfit |
| 10,00 g | Natriumlaurylethersulfat, 28%ige wässrige Lösung |
| 7,85 g | Ethanol |
| X g | Pyrazol der Formel (I) nach Tabelle 1 |
| Y g | Kuppler nach Tabelle 1 |
| 9,10g | Ammoniak, 25 %ige wässrige Lösung |
| ad 100,00 g | Wasser, entmineralisiert |

Unmittelbar vor der Anwendung werden 100 Gramm der vorstehenden Farbträgermasse mit 100 Gramm einer 6%igen wässrigen Wasserstoff-peroxidlösung vermischt und die erhaltene gebrauchsfertige Färbelösung in der erforderlichen Menge auf gebleichte Haare aufgetragen. Nach einer Einwirkzeit von 30 Minuten bei 40 °C wird das Haar mit einem Shampoo gewaschen, mit Wasser ausgespült und getrocknet. Die erhaltenen Farbnuancen und L*a*b*-Werte sind in Tabelle 1 zusammengefasst.

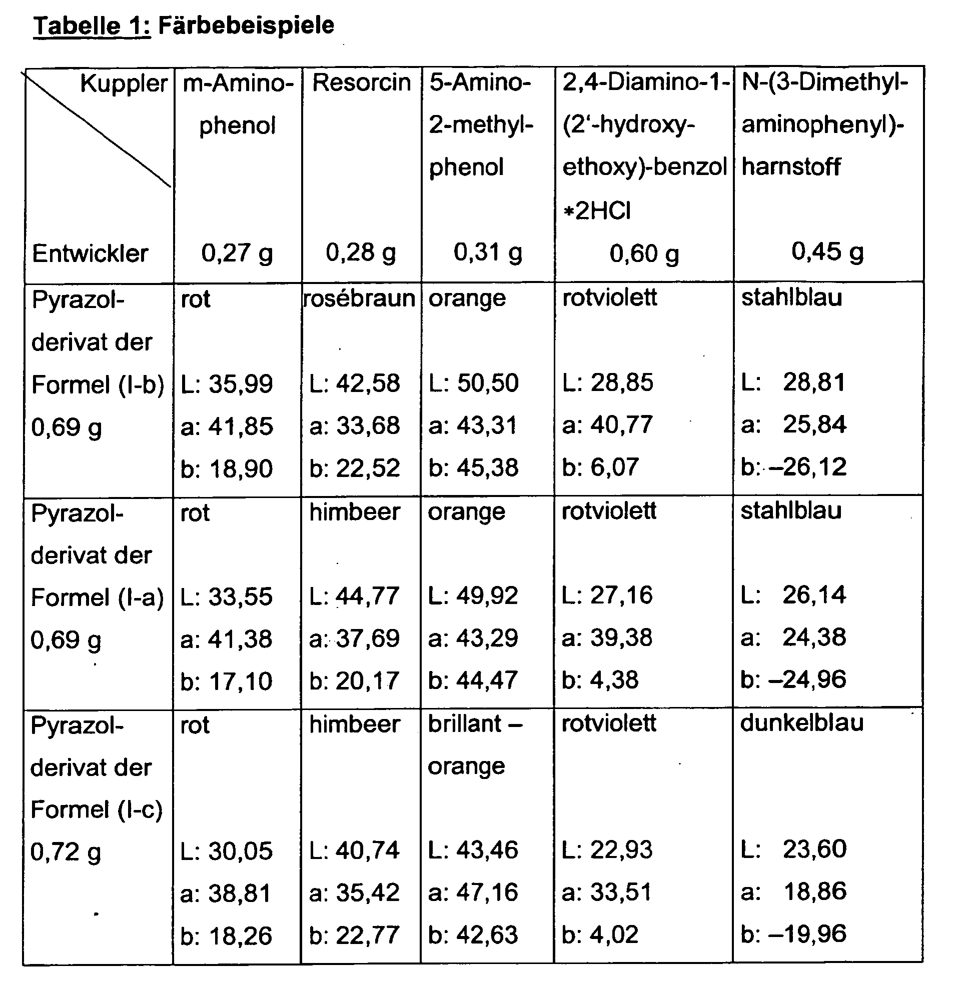

Die in den vorliegenden Beispielen angegebenen L*a*b*-Farbmesswerte wurden mit einem Farbmessgerät der Firma Minolta, Typ Chromameter CR-300, ermittelt.

Hierbei steht der L-Wert für die Helligkeit (das heißt je geringer der L-Wert ist, umso größer ist die Farbintensität), während der a-Wert ein Maß für den Rotanteil ist (das heißt je größer der a-Wert ist, umso größer ist der Rotanteil). Der b-Wert ist ein Maß für den Blauanteil der Farbe, wobei der Blauanteil umso größer ist, je negativer der b-Wert ist.

### Beispiel 7: Gelförmiges Oxidationshaarfärbemittel

| | |
|---|---|
| 15,00 g | Ölsäure |
| 3,00 g | Glycerin |
| 7,00 g | Isopropanol |
| 0,50 g | Ascorbinsäure |
| 0,40 g | Natriumsulfit |
| 0,40 g | Natriumhydroxid |
| 10,00 g | Ammoniak, 25%ige wässrige Lösung |
| 0,90 g | 1-(4-Aminophenyl)-4,5-diamino-1 H-pyrazol-sulfat(1:1) |
| 0,31 g | 1,4-Diamino-2-methyl-benzol-sulfat |
| 0,20 g | 1,4-Diamino-2-(2-hydroxyethyl)-benzol-sulfat |
| 0,10 g | 4-Amino-3-methyl-phenol |
| 0,46 g | 1,3-Diamino-4-(2-hydroxyethoxy)-benzol-dihydrochlorid |
| 0,33 g | 5-((2-Hydroxyethyl)amino)-2-methyl-phenol |
| 0,21 g | 3-Amino-phenol |
| ad 100,00 g | Wasser, entmineralisiert |

Unmittelbar vor der Anwendung werden 100 Gramm der vorstehenden Farbträgermasse (pH = 10,8) mit 100 Gramm einer 6%igen wässrigen Wasserstoff-peroxidlösung vermischt und das erhaltene gebrauchsfertige Oxidationshaarfärbemittel in der erforderlichen Menge auf zu 50 % ergrautes Humanhaar aufgetragen. Nach einer Einwirkzeit von 30 Minuten bei 40 °C wird das Haar mit einem Shampoo gewaschen, mit Wasser gespült und getrocknet. Das Haar ist schwarz mit einem Auberginereflex gefärbt.

### Beispiel 8: Oxidationsfärbemittel

| | |
|---|---|
| 0,30 g | Ascorbinsäure |
| 0,40 g | Natriumsulfit |
| 10,00 g | Natriumlaurylethersulfat, 28%ige wässrige Lösung |
| 7,85 g | Ethanol |
| 0,72 g | 1-(4-Aminophenyl)-4,5-diamino-1H-pyrazol-sulfat(1:1) |
| 0,31 g | 5-Amino-2-methylphenol |
| 9,10 g | Ammoniak, 25 %ig |
| ad 100,00 g | Wasser, entmineralisiert |

Unmittelbar vor der Anwendung werden 100 Gramm der vorstehenden Farbträgermasse mit 100 Gramm einer 6%igen wässrigen Wasserstoff-peroxidlösung vermischt und das erhaltene gebrauchsfertige Oxidationshaarfärbemittel in der erforderlichen Menge auf gebleichtes Büffelhaar, das einem stärker geschädigten Humanhaar entspricht, aufgetragen. Nach einer Einwirkzeit von 30 Minuten bei 40 °C werden die Haare mit einem Shampoo gewaschen, mit Wasser ausgespült und getrocknet. Es wird eine orange-rote Färbung erhalten.

Alle Prozentangaben in der vorliegenden Anmeldung stellen, soweit nicht anders angegeben, Gewichtsprozente dar.

## Patentansprüche

1. N-Aryl-4,5-diamino-pyrazol der Formel (I) oder dessen physiologisch verträglichen Salze mit organischen oder anorganischen Säuren, 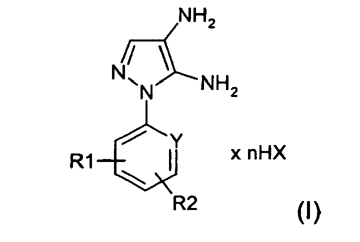 wobei
**R1** und **R2** unabhängig voneinander gleich einer geradkettigen oder verzweigten C1-C6-Alkylgruppe, einer Hydroxygruppe, einer geradkettigen oder verzweigten C1-C6-Monohydroxyalkylgruppe, einer geradkettigen oder verzweigten C3-C6-Dihydroxyalkylgruppe, einer geradkettigen oder verzweigten C1-C6-Alkoxygruppe, einer geradkettigen oder verzweigten C1-C6-Hydroxyalkoxygruppe, einer geradkettigen oder verzweigten C3-C6-Dihydroxyalkoxygruppe, einer Aminogruppe, einer C1-C4-Monoalkylaminogruppe, einer Di(C1-C4)-alkylaminogruppe, einer C1-C4-Aminoalkylgruppe, einem Halogenatom, einer Difluormethylgruppe oder einer Trifluormethylgruppe ist;
**Y** eine **C-R3-**Gruppe darstellt, wobei **C** ein Kohlenstoffaton des aromatischen Ringes ist und **R3** gleich einem Wasserstoffatom, einer geradkettigen oder verzweigten C2-C6-Hydroxyalkoxygruppe oder einer geradkettigen oder verzweigten C2-C6-Alkoxyalkoxygruppe ist; und
**X** für einen Säurerest steht und **n** einen Wert von 0 bis 3 hat;
oder **Y** eine **CH-**Gruppe darstellt und **R1** gleich einem Wasserstoffatom ist und **R2** eine Aminogruppe (in 4-Stellung am Benzolring) darstellt und **X** für eine HSO4-Gruppe steht und **n** gleich 1 ist.

2. N-Aryl-4,5-diamino-pyrazol nach Anspruch 1, **dadurch gekennzeichnet, dass** in Formel (I) gilt: **R1** und **R2** stehen unabhängig voneinander für eine Methylgruppe, eine Ethylgruppe, eine Isopropylgruppe, eine Aminogruppe oder eine Methoxygruppe, und **Y** steht für eine **CH**-Gruppe; oder es gilt: **Y** steht für eine **CH**-Gruppe und **R1** ist gleich einem Wasserstoffatom und **R2** stellt eine Aminogruppe (in 4-Stellung am Benzolring) dar und **X** steht für eine HSO4-Gruppe und **n** ist gleich **1.**

3. N-Aryl-4,5-diamino-pyrazol nach Anspruch 1 oder 2, **dadurch gekennzeichnet, dass** es ein Salz der Schwefelsäure, Salzsäure, Zitronensäure oder Weinsäure ist.

4. N-Aryl-4,5-diamino-pyrazol nach einem der Ansprüche 1 bis 3, **dadurch gekennzeichnet, dass** es ausgewählt ist aus 1-(2,4-Dimethylphenyl)-4,5-diamino-1H-pyrazol-dihydrochlorid, 1-(2,5-Dimethylphenyl)-4,5-diamino-1H-pyrazol-dihydrochlorid und 1-(4-Aminophenyl)-4,5-diamino-1H-pyrazol-sulfat(1:1).

5. Mittel zur oxidativen Färbung von Keratinfasern, **dadurch gekennzeichnet, dass** es mindestens ein N-Aryl-4,5-diamino-pyrazol nach einem der Ansprüche 1 bis 4 enthält.

6. Mittel nach Anspruch 5, **dadurch gekennzeichnet, dass** es das N-Aryl-4,5-diamino-pyrazol in einer Menge von 0,005 bis 20 Gewichtsprozent enthält.

7. Mittel nach Anspruch 5 oder 6, **dadurch gekennzeichnet, dass** es zusätzlich weitere Farbkomponenten aus der Gruppe bestehend aus Entwicklersubstanzen, Kupplersubstanzen, 4-(2,5-Diamino-benzylamino)-anilin, 3-(2,5-Diamino-benzylamino)-anilin, natürlichen Farbstoffen, naturidentischen Farbstoffen und synthetischen direktziehenden Farbstoffen enthält.

8. Gebrauchsfertiges Mittel zum oxidativen Färben von Keratinfasern, **dadurch gekennzeichnet, dass** es durch Vermischen eines Färbemittels nach einem der Ansprüche 5 bis 7 mit einem Oxidationsmittel in einem Gewichtsverhältnis von 5:1 bis 1:3 erhalten wird.

9. Mittel nach Anspruch 8, **dadurch gekennzeichnet, dass** das gebrauchsfertige Oxidationsfärbemittel einen pH-Wert von 3 bis 11 aufweist.

10. Mittel nach einem der Ansprüche 5 bis 9, **dadurch gekennzeichnet, dass** es ein Haarfärbemittel ist.

## Claims

1. N-Aryl-4,5-diaminopyrazole of the formula (I) or physiologically compatible salts thereof with organic or inorganic acids, 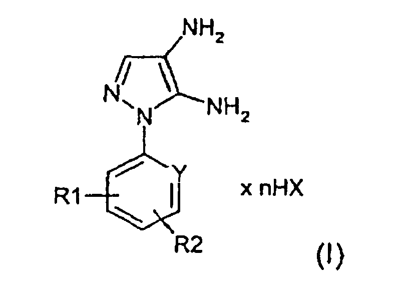 where
**R1** and **R2,** independently of one another, are a straight-chain or branched C1-C6-alkyl group, a hydroxy group, a straight-chain or branched C1-C6-monohydroxyalkyl group, a straight-chain or branched C3-C6-dihydroxyalkyl group, a straight-chain or branched C1-C6-alkoxy group, a straight-chain or branched C1-C6-hydroxyalkoxy group, a straight-chain or branched C3-C6-dihydroxyalkoxy group, an amino group, a C1-C4-monoalkylamino group, a di(C1-C4)alkylamino group, a C1-C4-aminoalkyl group, a halogen atom, a difluoromethyl group or a trifluoromethyl group;
**Y** is a **C-R3** group, where **C** is a carbon atom of the aromatic ring and **R3** is a hydrogen atom, a straight-chain or branched C2-C6-hydroxyalkoxy group or a straight-chain or branched C2-C6-alkoxyalkoxy group; and
**X** is an acid radical and n has a value from 0 to 3;
or **Y** is a **CH** group and **R1** is a hydrogen atom and **R2** is an amino group (in position 4 on the benzene ring) and **X** is an HS04 group and n is 1.

2. N-Aryl-4,5-diaminopyrazole according to Claim 1, **characterized in that**, in formula (I): **R1** and **R2,** independently of one another, are a methyl group, an ethyl group, an isopropyl group, an amino group or a methoxy group, and **Y** is a **CH** group; or: **Y** is a **CH** group and **R1** is a hydrogen atom and **R2** is an amino group (in position 4 on the benzene ring) and **X** is an HS04 group and **n** is 1.

3. N-Aryl-4,5-diaminopyrazole according to Claim 1 or 2, **characterized in that** it is a salt of sulphuric acid, hydrochloric acid, citric acid or tartaric acid.

4. N-Aryl-4,5-diaminopyrazole according to one of Claims 1 to 3, **characterized in that** it is chosen from 1-(2,4-dimethylphenyl)-4,5-diamino-1H-pyrazole dihydrochloride, 1-(2,5-dimethylphenyl)-4,5-diamino-1H-pyrazole dihydrochloride and 1-(4-aminophenyl)-4,5-diamino-1H-pyrazole sulphate (1:1) .

5. Agent for the oxidative dyeing of keratin fibres, **characterized in that** it comprises at least one N-aryl-4,5-diaminopyrazole according to one of Claims 1 to 4.

6. Agent according to Claim 5, **characterized in that** it comprises the N-aryl-4,5-diaminopyrazole in an amount of from 0.005 to 20 per cent by weight.

7. Agent according to Claim 5 or 6, **characterized in that** it additionally comprises further colour components from the group of developer substances, coupler substances, 4-(2,5-diaminobenzyl-amino)aniline, 3(2,5-diaminobenzylamino)aniline, natural dyes, nature-identical dyes and synthetic direct dyes.

8. Ready-to-use agent for the oxidative dyeing of keratin fibres, **characterized in that** it is obtained by mixing a colorant according to one of Claims 5 to 7 with an oxidizing agent in a weight ratio of from 5:1 to 1:3.

9. Agent according to Claim 8, **characterized in that** the ready-to-use oxidation colorant has a pH of from 3 to 11.

10. Agent according to one of Claims 5 to 9,
**characterized in that** it is a hair colorant.

## Revendications

1. N-aryl-4,5-diaminopyrazole de formule (I) ou sels physiologiquement acceptables de celui-ci avec des acides organiques ou minéraux 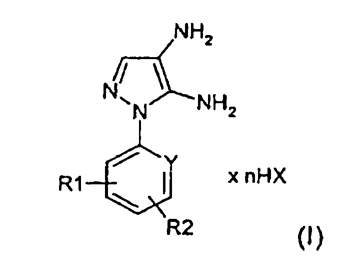 formule dans laquelle
**R1** et **R2** représentent indépendamment un groupe alkyle en C₁-C₆ à chaîne droite ou ramifiée, un groupe hydroxy, un groupe monohydroxyalkyle en C₁-C₆ à chaîne droite ou ramifiée, un groupe dihydroxyalkyle en C₃-C₆ à chaîne droite ou ramifiée, un groupe alcoxy en C₁-C₆ à chaîne droite ou ramifiée, un groupe hydroxyalcoxy en C₁-C₆ à chaîne droite ou ramifiée, un groupe dihydroxy-alcoxy en C₃-C₆ à chaîne droite ou ramifiée, un groupe amino, un groupe monoalkylamino en C₁-C₄, un groupe dialkyl(C₁-C₄)amino, un groupe aminoalkyle-C₁-C₄, un atome d'halogène, un groupe difluorométhyle ou un groupe trifluorométhyle ;
**Y** représente un groupe **C-R3, C** étant un atome de carbone du cycle aromatique et **R3** représentant un atome d'hydrogène, un groupe hydroxyalcoxy en C₂-C₆ à chaîne droite ou ramifiée ou un groupe alcoxyalcoxy en C₂-C₆ à chaîne droite ou ramifiée ; et
**X** représente un reste d'acide et **n** a une valeur de 0 à 3 ;
ou **Y** représente un groupe **CH** et **R1** est un atome d'hydrogène et **R2** représente un groupe amino (en position 4 sur le cycle benzénique) et **X** représente un groupe HSO₄ et **n** est égal à 1.

2. N-aryl-4,5-diaminopyrazole selon la revendication 1, **caractérisé en ce que** dans la formule (I) : **R1** et **R2** représentent, indépendamment l'un de l'autre, un groupe méthyle, un groupe éthyle, un groupe isopropyle, un groupe amino ou un groupe méthoxy, et **Y** représente un groupe **CH ;** ou bien : **Y** représente un groupe **CH** et **R1** représente un atome d'hydrogène et **R2** représente un groupe amino (en position 4 sur le cycle benzénique) et **X** représente un groupe HSO₄ et **n** est égal à 1.

3. N-aryl-4,5-diaminopyrazole selon la revendication 1 ou 2, **caractérisé en ce qu'**il s'agit d'un sel de l'acide sulfurique, chlorhydrique, citrique ou tartrique.

4. N-aryl-4,5-diaminopyrazole selon l'une quelconque des revendications 1 à 3, **caractérisé en ce qu'**il est choisi parmi le dichlorhydrate de 1-(2,4-diméthylphényl)-4,5-diamino-1H-pyrazole, le dichlorhydrate de ●1-(2,5-diméthylphényl)-4,5-diamino-1H-pyrazole et le sulfate (1:1) de 1-(4-aminophényl)-4,5-diamino-1H-pyrazole.

5. Composition pour la teinture par oxydation de fibres de kératine, **caractérisée en ce qu'**elle contient au moins un N-aryl-4,5-diaminopyrazole selon l'une quelconque des revendications 1 à 4.

6. Composition selon la revendication 5, **caractérisée en ce qu'**elle contient le N-aryl-4,5-diaminopyrazole en une quantité de 0,005 à 20 % en poids.

7. Composition selon la revendication 5 ou 6,
**caractérisée en ce qu'**elle contient en outre d'autres composants de teinture choisis dans le groupe constitué par des développeurs, des coupleurs, la 4-(2,5-diamino-benzylamino)aniline, la 3-(2,5-diamino-benzylamino)-aniline, des colorants naturels, des colorants ressemblant aux colorants naturels et des colorants directs synthétiques.

8. Composition prête à l'emploi, pour la teinture par oxydation de fibres de kératine, **caractérisée en ce qu'**elle est obtenue par mélange d'une composition colorante selon l'une quelconque des revendications 5 à 7, avec un oxydant en un rapport pondéral de 5:1 à 1:3.

9. Composition selon la revendication 8, **caractérisée en ce que** la composition de teinture par oxydation prête à l'emploi présente un pH de 3 à 11.

10. Composition selon l'une quelconque des revendications 5 à 9, **caractérisée en ce qu'**elle est une composition de teinture pour cheveux.
